# EUROPEAN PATENT APPLICATION

(11) **EP 4 715 066 A1**
(43) Date of publication of application: **25.03.2026**
(21) Application number: 24806725.8
(22) Date of filing: 17.05.2024
(51) Int. Cl.: C12Q 1/6883

(54) **PANEL OF MICRORNAS AS BIOMARKERS OF NON-SEGMENTAL VITILIGO**

(30) Priority: 17.05.2023 ES 202330384
(71) Applicant: Servicio Andaluz de Salud, 41071 Sevilla (ES); Universidad de Granada, 18071 Granada (ES)
(72) Inventor: RUANO RUIZ, Juan, 14004 Córdoba Córdoba (ES); ISLA TEJERA, Beatriz, 14004 Córdoba Córdoba (ES); GAY MIMBRERA, Jesús, 14004 Córdoba Córdoba (ES); GÓMEZ ARIAS, Pedro Jesús, 14004 Córdoba Córdoba (ES); LIÑARES BLANCO, José, 18071 Granada Granada (ES); AGUILAR LUQUE, Macarena, 14004 Córdoba Córdoba (ES)
(74) Representative: San Martin Alarcia, Esther
(86) International application number: PCT/ES2024/070299
(87) International publication number: WO 2024/236215

(57) **Abstract**

The invention relates to a panel of 6 microRNAs and the use thereof as biomarkers in the prognosis and/or diagnosis of non-segmental vitiligo according to their expression levels in damaged skin or plasma. The invention also relates to methods for obtaining useful data for the diagnosis and/or prognosis of non-segmental vitiligo, as well as kits to carry out said methods.

## Description

### TECHNICAL FIELD

The present invention falls within the field of medicine, specifically the field of medical prognosis and diagnosis, and even more specifically relates to the use of microRNAs (miRNAs) for the diagnosis and prognosis of non-segmental vitiligo.

### BACKGROUND OF THE INVENTION

Vitiligo is an autoimmune skin disease characterized by the development of hypopigmented or achromic macules. These macules can be presented with different distributions on the skin, allowing vitiligo to be classified into two main subtypes: segmental vitiligo, which affects unilateral segments relative to the midline of the body, and non-segmental vitiligo, which is characterized by the presence of these macules mainly around the orifices, on the extremities, and on bony prominences, areas where there is a greater risk of skin trauma.

Non-segmental vitiligo occurs with equal frequency in individuals regardless of race or sex and can develop at any age, although it is more common in the first two decades of life, manifesting before the age of 20 in 50% of cases. The etiology of the disease is unknown, and the biological mechanisms related to its development include selective apoptosis of melanocytes due to a chronic inflammatory state of the skin involving cytotoxic T cells, *natural killer* cells, Th1, Th2, and Th17 lymphocytes, and IL23-producing Langerhans cells, as well as metabolic pathways such as WNT and JAK/STAT.

The progression of the disease is unpredictable and is characterized by periods of disease inactivity interspersed with slow or rapid depigmentation, which often indicates a worse prognosis and a longer course of treatment. To date, there are no methods to predict the progression to these more aggressive forms of non-segmental vitiligo.

Several drugs targeting severe cases are currently in clinical trials **(1),** so it would be interesting to know which patients are at greater risk of progressing to more severe forms of the disease and, in such cases, to assess whether early therapies would be able to prevent such progression.

The criteria for defining disease activity are based mainly on studies conducted with small groups of patients, with the limitations that this implies. The different scales used to estimate the severity of non-segmental vitiligo are based on the calculation of the affected body surface area. To date, several scales have been used, including:
- **Vitiligo Area Scoring Index (VASI),** which uses a formula that includes the entire body (with a possible range of 0 to 100), which is divided into six separate and mutually exclusive areas (head and neck, hands, upper limbs excluding hands, trunk, lower limbs excluding feet, and feet), indicating the percentage of vitiligo affectation in each area in the form of "hand units" and the degree of depigmentation within these affected hand units. The measurement is performed by clinical staff.
- **Self Assessed Vitiligo Area Scoring Index (SAVASI)** uses the same basic principles as VASI, adapted for use by the patient, for which it includes an illustrated document with a descriptive atlas of patient photographs.
- **Vitiligo Noticeability Scale (VNS)** allows patients to assess how visible their vitiligo lesions are after therapy, based on examples of images taken before and after treatment, rather than on changes observed in their own vitiligo with therapy. There is no evidence to support the reliability of this instrument.
- **Vitiligo European Task Force Assessment (VETFa)** divides the body into five areas (head/neck, hands and feet, trunk, arms, legs) and assesses in them the extent or percentage of vitiligo affectation estimated using the "Wallace rule of nines" and provides a classification based on the severity of skin and hair depigmentation (stage 0: normal pigmentation; stage 1: incomplete pigmentation; stage 2: complete depigmentation; stage 3: partial hair whitening) and gives higher scores when the hair is affected. It also assesses disease progression by detecting depigmentation in early stages using a Wood's lamp. However, VETFa only measures one lesion from each region of the body, losing data from the entire affected BSA.
- **Vitiligo Extent Score (VES),** it was developed to simplify scales such as VASI or VETFa. A panel of standard images of different degrees of affectation in different parts of the body is used as a reference, from which clinical staff choose those that best represent the patient's skin lesions and then the percentage of depigmented area is calculated.
- **Self Assessment Vitiligo Extent Score (SA-VES),** it is a simplified version of the VES tool for its use by patients.

These tools use measurement methods that are not exempt from limitations. The VETFa tools use "Wallace rule of nines" to estimate the extent of the affected areas, which assigns a percentage of multiples of 9 to each part of the body divided into eight zones, adding up to 100%. This method has significant limitations when applied to people with obesity. VASI and SAVASI use "hand units" that depend on the ability of clinical staff to mentally add up all the spots to obtain the equivalent number of palms. VES and SA-VES use templates to assess the extent of vitiligo, with the difficulty of mentally comparing the template with the extent of the disease in patients.

In addition, all these measurement tools require photographs to be taken in order to assess the disease and have significant limitations when it comes to taking measurements in small affected areas.

Attempts have been made to assess the progression of this disease; however, only a qualitative assessment of the speed of progression has been achieved, comparing the days on which lesions increase in small groups of patients compared to others, with no quantitative and standardized measure having been identified to date.

Other scales allow for the assessment of other processes related to the disease, such as:
- **Koebner's Phenomenon in Vitiligo Score (K-VSCOR),** a diagnostic test and mathematical model used to identify patients with a high probability of developing the Koebner phenomenon. The Koebner phenomenon refers to the fact that vitiligo lesions can appear on healthy skin when lesions occur on it.
- **Potential Repigmentation Index (PRI),** which measures the individual capacity of each lesion to repigment and takes into account the total number of lesions. It classifies lesions into four clinical types of vitiligo: A, B, C, and D, which presumably correspond to the remaining follicular and epidermal reservoirs of melanocytes and precursors.
- **Vitiligo Signs of Activity Score (VSAS)** allows the degree of vitiligo activity to be assessed by evaluating the number of locations with at least one sign of disease activity.
- **Vitiligo Disease Activity (VIDA)** is a 6-point scale for assessing disease activity and is intended to help evaluate the effectiveness of interventions to stop and reverse the degree of depigmentation. The score is based on the individual's own opinion of current disease activity over time. Active vitiligo involves the expansion of existing lesions or the appearance of new lesions. The rating is as follows: VIDA score +4 - Activity lasting 6 weeks or less; +3 - Activity lasting 6 weeks to 3 months; +2 - Activity lasting 3-6 months; +1 - Activity lasting 6-12 months; 0 - Stable for 1 year or more; and -1 - Stable with spontaneous repigmentation for 1 year or more. A low VIDA score indicates less activity.

In summary, the most efficient tool for measuring non-segmental vitiligo is currently VES, including SA-VES, and it has many limitations, including inferior performance in patients with extensive affectation. The severity of the disease can be measured with the VASI tool because it combines the extent and degree of depigmentation. However, there is no evidence to recommend the use of SAVASI, considering the doubts about its sensitivity. VETFa does not improve VES nor is it capable of assessing the progression or stage of the disease, while VNS and K-VSCOR lack evidence of reliability.

None of these scales provide a validated and objective quantitative tool that allows for the joint and unambiguous assessment of the degree of disease progression, the speed of spread, and the degree of response to treatment or reversal of depigmentation. Therefore, it is necessary to identify new markers based on large-scale prospective studies with long-term follow-up that allow for the design of early intervention strategies.

Other processes related to non-segmental vitiligo, such as the Koebner phenomenon, trichromic lesions or confetti depigmentation disease, have been described as clinical markers. The latter is characterized by presenting lesions measuring one to three millimeters, which can be found in vitiligo, but are more characteristic of chemical leukodermas (skin discoloration) or tuberous sclerosis. Its presence is associated with an unfavorable prognosis, as it carries a higher risk of disease progression and poor therapeutic response (2).

A group of proteins related to skin pigmentation processes belonging to the WNT signaling pathway described in the patent WO2016097035A1 have also been proposed as biomarkers: LEF (Lymphoid enhancer-binding factor), TCF (DNA-binding transcription factor), MITF (Microphthalmia-associated transcription factor), DCT (Dopachrome tautomerase or dopachrome delta-isomerase, tyrosine-related protein 2), PAX3 (transcription factor 3 of the paired box (PAX) family), and transcription factor BRn2. They are related to methods of diagnosis and treatment of pigmentation disorders, including vitiligo.

It is also known that a wide variety of miRNAs are associated with genetic polymorphisms (e.g., miR-196a-2 rs11614913), immune response (e.g., miR-133b, miR-224-3p, miR-4712-3p, miR-3940-5p, miR -21-5p), oxidative stress (e.g., miR-135a, miR-9, miR-34a, miR-183, miR-184, miR-1, miR-25, miR-211, miR-383, miR-577 , miR-421) and melanocyte functions (e.g., miR-434-5p, miR-330-5p, miR-137, miR-148, miR-145, miR-155, miR-203, miR-125, miR-377, miR-2909, miR-200c, hsa-miR-149-5p) involved in the pathological mechanism of vitiligo.

miRNAs are small endogenous non-coding RNAs approximately 18-25 nucleotides in length, capable of modulating the expression of complementary messenger RNAs. They negatively regulate (inhibit) gene expression either by inhibiting translation or by cleaving the messenger RNA. Therefore, miRNAs are post-transcriptional regulators that bind to complementary sequences of mRNA transcripts by pairing with the untranslated region (3'-UTR), which generally results in repression of translation or degradation of the target, leading to gene silencing.

They are involved in the regulation of most molecular processes, and there is consistent scientific evidence regarding their relationship with various metabolic processes. Circulating miRNAs can be detected in the bloodstream, where they exhibit high stability and reproducibility. Circulating miRNAs can provide clinical information about pathophysiological conditions, enabling early diagnosis and study of the progression of various diseases, and are therefore proposed as potential biomarkers.

Under a standard nomenclature system, experimentally confirmed miRNAs are assigned names as follows: the prefix "mir," followed by a hyphen and a number. The lowercase "mir-" refers to the pre-miRNA, while an uppercase "miR-" refers to the mature form. miRNAs with nearly identical sequences (except for one or two nucleotides) are annotated with an additional lowercase letter, e.g., miR-99a. Pre-miRNAs that lead to 100% identical mature miRNAs but are located at different sites in the genome are indicated with an additional hyphenated number suffix. The species of origin can be designated with a three-letter prefix, where hsa corresponds to a human miRNA (*Homo sapiens*). In the context of this document, where all individualized miRNAs are human miRNAs, the prefix "hsa-" is sometimes omitted. When two mature miRNAs originate from opposite arms of the same pre-miRNA, they are denoted with a suffix -3p or -5p, such as miR-142-3p. When relative expression levels are known, an asterisk after the name indicates a miRNA expressed at low levels relative to the miRNA on the opposite arm of a hairpin. miRNA sequences can be accessed at http://www.mirbase.org.

### BRIEF DESCRIPTION OF THE INVENTION

In the present invention, 6 miRNAs have been identified that act as biomarkers because they are differentially expressed in both damaged tissue and peripheral blood in patients with non-segmental vitiligo. Some of the deregulated miRNAs that make up this panel have been previously described in other dermatological diseases, but very few previous studies have analyzed the role of miRNAs in the peripheral blood of patients with non-segmental vitiligo, and those that address this aspect yield contradictory results. Of the 6 miRNAs proposed in the invention panel, none has been previously described as associated with non-segmental vitiligo.

The present invention **proposes a panel of microRNAs (miRNAs),** whose expression is deregulated both in tissue samples isolated from skin lesions caused by vitiligo and in the plasma of patients with non-segmental vitiligo compared to healthy individuals, as biomarkers of the disease. Differences in the expression of this group of miRNAs result in the dysregulation of several immunological and metabolic pathways related to disease severity.

The 6 miRNAs that make up the panel of the invention for the diagnosis and prognosis of non-segmental vitiligo, or **miRNAs of the invention,** are as follows: miR-16-5p, miR-92a-3p, miR-486-5p, miR-221-3p, miR-23a-3p and miR-652-3p.

All of them are differentially expressed in the damaged skin and plasma of patients with non-segmental vitiligo compared to controls, in whom no significant changes were found.

The expression levels of the miRNAs of the invention are determined in samples of damaged skin or blood previously obtained from patients. Therefore, a predictive model based on the determination of the expression levels of 6 miRNAs in damaged skin or plasma is proposed below.

This disease classification method has the advantage of being an objective and reproducible method compared to current scales based on images or photographs.

In turn, by measuring the expression levels of the miRNAs of the invention over time, this method is capable of monitoring the therapeutic response to treatment of the disease with various drugs.

Therefore, the method of the invention can also be used as a molecular predictor of therapeutic response to different treatments for non-segmental vitiligo. Thanks to the miRNAs proposed in the present invention and their differential expression in patients with non-segmental vitiligo, it is possible to carry out an assessment and repositioning of drugs capable of reversing this state of dysregulation. This assessment is performed using the Cmaps and LINCS tools. The Broad Institute's Connectivity Map (CMap) project uses the library of integrated network-based cellular signatures (LINCS) and allows functional hypotheses to be generated from cellular signatures representing systematic perturbations with genetic perturbogens (reflecting protein function) and pharmacological perturbogens (reflecting small molecule function).

Once the lists of potential drugs on the market or in development or molecules in the experimental phase contained in these databases were obtained and after this analysis, **121 drugs** potentially capable of reversing the state of dysregulation in the expression of the miRNAs of the invention were finally selected. This is possible through GeneCodis, which uses the molecular signatures found to find similarities with other signatures produced by these perturbogens of interest and stored in the aforementioned databases. Finally, those results obtained with an FDR value < 0.05 and by the *relative enrichment* value in descending order were selected.

Of this final list, **37.2%** of the drugs that showed a potential ability to reverse the profiles of gene dysregulation by miRNAs in non-segmental vitiligo were **kinase inhibitors** [led by PI3K inhibitors (12.4%; alpelisib, pictilisib, PI-103, dactolisib, LY-294002, AS-605240, AZD-6482, ZSTK-474, TG100-115, PIK-75, KU-0060648, wortmannin, rigosertib, GSK-1059615, AS-604850); multi-kinases (dovitinib, sorafenib, tivozanib, sunitinib, pazopanib, regorafenib, imatinib, dasatinib, ENMD-2076, GTP-14564, talazoparib, nilotinib, masitinib); IKK (BMS-345541, TPCA-1, IKK-2-inhibitor-V, IKK-16); JAK2 (fedratinib); VEGF (SU1498, semaxanib, brivanib, Oxindole-I); VEGFR2 (cediranib, linifanib); HER/Erb2 (neratinib, CP-724714, tyrphostin-AG-825); PCK (bisindolylmaleimide-ix, SA-792987, 4,5,6,7-tetrabromobenzotriazole); Ras (manumycin-a); Raf (HG-6-64-01, GW-5074); p38 MAPK (SB-239063, SB-202190, adezmapimod); checkpoint kinase (SB-218078, AZD-7762); mTor kinase (everolimus, temsirolimus); MEK-1 (PD-98059, U-0126), FLT3 (tandutinib, midostaurin, quizartinib); CDK4 and CDK6 (abemaciclib, palbociclib, ribociclib); CDK9 (alvocidib)].

Other drugs identified were phosphodiesterase inhibitors (2.48%; cilomilast, sildenafil, YM-976), BCL-2 inhibitors (5.8%; ABT-737, navitoclax, TW-37, BH3I-1, HA-14-1, obatoclax, venetoclax), microtubule inhibitors (0.76%; eribulin, ixabepilone), survivin inhibitors (0.38%; YM-155), sirtuin inhibitors (0.38%; splitomycin), monoclonal antibodies [anti-VEGF (2.48%; aflibercept, ranibizumab, bevacizumab), anti-EGFR (1.65%; panitumumab, cetuximab), anti-HER2 (1.65%; pertuzumab, trastuzumab) and anti-TNF (0.83%; adalimumab)], antioxidants (8.26%; embelin, epigallocatechin, piceatannol, gossypol, edaravone, resveratrol, guggulsterone, curcumin, isoliquiritigenin, pentoxifylline), anti-inflammatories (9.09%; dapsone, flufenamic acid, aspirin, celecoxib, ibuprofen, dexamethasone, mesalazine, sulfasalazine, corticosteroids), antimicrobials (3.31%; mepacrine, pyrimethamine, triclabendazole, triclosan), hormone regulators (3.31%; danazol, levonorgestrel, gestrinone, tibolone), epigenetic modifiers (1.65%; zebularine, decitabine), p53 expression restorers (2.48%; pifithrin-alpha, prima-1-met, pifithrin-mu), PPARG receptor agonists (7.7%; troglitazone), and proteasome inhibitors (0.38%; carfilzomib).

13.07% were substances with toxic effects, such as chemotherapeutics, or were in very early stages of development and were therefore not considered a priority.

Therefore, the present invention also develops a method capable of predicting the therapeutic response of different treatments, either under development or already approved for clinical use, against severe non-segmental vitiligo.

Finally, the present invention describes a kit for RT-PCR analysis of the 6 human miRNAs using samples of damaged skin in order to identify patients with vitiligo.

### DETAILED DESCRIPTION OF THE INVENTION

A **first aspect** of the invention relates to the *in vitro* use of the expression levels of one or more of the following miRNAs: miR-16-5p, miR-92a-3p, miR-486-5p, miR-221-3p, miR-23a-3p, and/or miR-652-3p as biomarkers for predicting and/or diagnosing non-segmental vitiligo. In a preferred embodiment, the expression levels of these biomarkers are used simultaneously.

**Table 1: miRNA sequences of the invention.**

| **Name** | **SEQ ID No.** | **Sequence (5'-3')** | **Accession number** |
|---|---|---|---|
| hsa-miR-16-5p | 1 | uagcagcacguaaauauuggcg | MIMAT0000069 |
| hsa-miR-92a-3p | 2 | uauugcacuugucccggccugu | MIMAT0000092 |
| hsa-miR-486-5p | 3 | uccuguacugagcugccccgag | MIMAT0002177 |
| hsa-miR-221-3p | 4 | agcuacauugucugcuggguuuc | MIMAT0000278 |
| hsa-miR-23a-3p | 5 | aucacauugccagggauuucc | MIMAT0000078 |
| hsa-miR-652-3p | 6 | aauggcgccacuaggguugug | MIMAT0003322 |

Another **aspect** of the invention relates to an expression profile of the miRNAs in **Table 1,** both individually and in any combination thereof, suitable for diagnosing non-segmental vitiligo, hereinafter referred to as the expression profile of biomarkers of the invention. Preferably, the expression profile is composed of all the miRNAs in Table 1 simultaneously.

### METHODS OF THE INVENTION

Another **aspect** of the invention relates to an *in vitro* method for obtaining useful data for diagnosing and/or predicting severe non-segmental vitiligo, comprising measuring the expression levels of one or more of the miRNAs in **Table 1** in a previously isolated biological sample. Preferably, the expression levels of all the miRNAs in Table 1 are measured simultaneously.

In a preferred embodiment of this aspect of the invention, the biological sample is selected from damaged skin or whole blood, serum, or plasma, preferably plasma.

Another **aspect** of the present invention relates to an *in vitro* method for diagnosing and/or predicting severe non-segmental vitiligo comprising:
a) measuring the expression levels of one or more of the miRNAs in Table 1 in a previously isolated damaged skin tissue sample,
b) comparing the values obtained with those of a reference sample obtained from healthy control individuals, and
c) classifying the individual in the group of individuals suffering from non-segmental vitiligo when the miRNA(s) in step a) are differentially down-expressed compared to samples from healthy control individuals.

Preferably, in step a), the expression levels of all miRNAs in Table 1 are measured simultaneously.

Another **aspect** of the present invention relates to an *in vitro* method for diagnosing and/or predicting non-segmental vitiligo comprising:
a) measuring the expression levels of all the miRNAs in Table 1 in a previously isolated blood, serum, or plasma sample,
b) comparing the values obtained with those of a reference sample obtained from healthy control individuals, and
c) classifying the individual in the group of individuals suffering from severe non-segmental vitiligo when the miRNAs in step a) hsa.miR.16.5p, hsa.miR.486.5p and hsa.miR.92a.3p are differentially down-expressed and hsa.miR.652.3p, hsa.miR.221.3p and hsa.miR.23a.3p are differentially over-expressed compared to samples from healthy control individuals.

*In vitro* determination of expression levels is performed using any of the techniques known in the prior art. Preferably, the techniques are selected from the list consisting of a gene profiling method, such as a microarray, or a next-generation sequencing panel and/or a method comprising PCR, such as real-time PCR or RT-PCR; and/or Northern Blot; and/or an immunohistochemistry method; and/or an ELISA-based method.

A microarray is an array on a solid substrate (usually a glass slide or a thin silicon film cell) that analyzes large amounts of biological material, in this case a large number of different miRNAs or, preferably, their reverse DNA transcripts, which are detectable by specific probes immobilized on the solid substrate.

A Northern transfer involves the use of electrophoresis to separate RNA samples by size and subsequent detection with a hybridization probe complementary to (part of) the target sequence of the RNA of interest.

Quantification of miRNA expression is preferably performed by RT-PCR.

Reverse transcription PCR, or RT-PCR, allows the detection and amplification of RNA. RNA is reverse transcribed into complementary DNA (cDNA) using reverse transcriptase. The first step in RT-PCR is the synthesis of a DNA/RNA hybrid. Reverse transcriptase also has an RNase H function, which degrades the RNA portion of the hybrid. The single-stranded DNA molecule is then completed into cDNA by the reverse transcriptase activity of DNA-dependent DNA polymerase. From here, the standard PCR procedure is used to amplify the cDNA. The ability to reverse RNA into cDNA using RT-PCR has many advantages, as RNA is single-stranded and highly unstable, making it difficult to work with.

The method of the present invention can be applied to samples from individuals of any sex, i.e., male or female, and of any age.

In the method of the present invention, miRNA expression can be normalized, preferably in relation to the expression of another RNA molecule. There are well-known normalization methods in the prior art.

The invention provides a method for assigning a human subject to one of two groups: group 1, comprising subjects identifiable by the method of the invention, and group 2, representing the remaining subjects.

It is possible to provide personalized treatment to an individual depending on whether the individual is assigned to group 1 or group 2. Group 1 comprises subjects identifiable by the method of the invention as individuals suffering from non-segmental vitiligo, and group 2 represents the remaining subjects.

In **another embodiment of the method of the invention,** it is capable of predicting the therapeutic response of different treatments for non-segmental vitiligo, either in development or already approved for clinical use, based on their ability to reverse the state of dysregulation of the expression of the miRNAs of the invention.

Therefore, we are talking about the use of one or more miRNAs in **Table 1,** and preferably all of them simultaneously, in a method for predicting the therapeutic response of different treatments for non-segmental vitiligo.

In this regard, another embodiment of the method of the invention relates to the method for predicting the therapeutic response of a patient with non-segmental vitiligo to a treatment for non-segmental vitiligo comprising:
a) measuring the expression levels of one or more of the miRNAs in Table 1 in a biological sample previously isolated from a patient with non-segmental vitiligo prior to administration of the treatment for vitiligo,
b) measuring the expression levels of the same miRNAs as in step a) in a biological sample previously isolated from the same patient with non-segmental vitiligo after administration of the treatment for vitiligo,
c) comparing the values obtained in steps a) and b), and
d) classifying the individual in the group of individuals responding to treatment when the miRNA(s) from step b) are differentially expressed with respect to the samples from step a).

On the other hand, it also refers to the method for predicting the therapeutic response to a treatment for non-segmental vitiligo in patients with non-segmental vitiligo, which comprises:
a) measuring the expression levels of one or more of the miRNAs in Table 1 in biological samples previously isolated from a statistically significant number of patients with non-segmental vitiligo prior to the administration of the treatment for vitiligo,
b) measuring the expression levels of the same miRNAs as in step a) in a biological sample previously isolated from the same patients with non-segmental vitiligo after administration of the treatment for vitiligo,
c) comparing the values obtained in steps a) and b), and
d) classifying the treatment for non-segmental vitiligo as effective when the miRNA(s) from step b) are differentially expressed with respect to the samples from step a) in a statistically significant number of patients with non-segmental vitiligo undergoing treatment.

Preferably, in both methods, in both steps a) and b) the expression levels of all miRNAs in Table 1 are measured simultaneously.

Among the potential drugs that can be subjected to this assessment of therapeutic response, and therefore their suitability for the treatment of non-segmental vitiligo, kinase inhibitors stand out.

### KIT OR DEVICE OF THE INVENTION

**Another aspect** of the invention relates to a kit or device comprising at least one or more oligonucleotides capable of hybridizing with one or more of the miRNAs in **Table 1** under stringent conditions. Preferably, it comprises at least one or more oligonucleotides capable of hybridizing to all of the miRNAs in Table 1 simultaneously under stringent conditions.

It is preferred that said oligonucleotide(s) be capable of doing so under stringent conditions. In a preferred embodiment, one or more of said one or more oligonucleotides (preferably DNA) are further defined by the following probes or primers: hsa-miRXX-miRCURY LNA miRNA measured with syber, specific LNA^{™} PCR primers.

Astringency is a term used in hybridization experiments. Astringency reflects the degree of complementarity between the oligonucleotide and the nucleic acid (which in this case is the miRNA to be detected); the higher the astringency, the higher the percentage of homology between the probe and the nucleic acid bound to the filter. Experts in the field are well aware that temperature and salt concentrations have a direct effect on the results obtained. It is recognized that hybridization results are related to the number of degrees below the Tm (melting temperature) of the DNA in which the experiment is performed. Often, rigorous conditions are defined as washing with 0.1X SSC (sodium citrate saline) buffer at 65°C. (SSC is usually provided as a 20X stock solution, consisting of 3 M sodium chloride and 300 mM trisodium citrate (adjusted to pH 7.0 with HCl)).

In particular embodiments, the kit is selected from (a) a kit suitable for microarray analysis, (b) a kit suitable for PCR, (c) a kit suitable for Northern Blot, and (d) a kit suitable for immunoassay. Two or more of these embodiments may also be combined, such that the kit may comprise, for example, both (a) and (c).

It is also possible for the oligonucleotide(s) to be immobilized in spots on a surface (preferably solid). In one embodiment thereof, the kit comprises a microarray. An RNA microarray is an array on a solid substrate (usually a glass slide or a silicon thin film cell) that analyzes large amounts of different RNAs (in this case, miRNAs), which can be detected by specific probes immobilized at points on the solid substrate. Each spot contains a specific nucleic acid sequence, typically a DNA sequence, known as probes (or informers). While the number of spots is not limited, there is a preferred embodiment in which the microarray is adapted to the methods of the invention. In one embodiment, such a customized microarray comprises fifty spots or fewer, such as thirty spots or fewer, including twenty spots or fewer.

In the case of (b) a kit suitable for PCR, this PCR is typically real-time quantitative PCR (RQ-PCR), a sensitive and reproducible gene expression quantification technique. In this case, it is desirable that the kit additionally comprises a polyT oligonucleotide primer in addition to the kit oligonucleotide(s). The polyT oligonucleotide primer can be used together with the oligonucleotide(s) of the invention for priming PCR, after polyadenylation of the isolated miRNAs by methods known to those skilled in the art, such as the use of poly-(A) polymerase and ATP. These reagents may optionally be included in the kit.

A Northern blot involves the use of electrophoresis to separate RNA samples by size and subsequent detection with an oligonucleotide(s) (hybridization probe) complementary to (part of) the target sequence of the RNA of interest.

The kit or device of the invention may be used and the use is not particularly limited, although use in the method of the invention in any of its embodiments is preferred.

In another preferred embodiment of this aspect of the invention, the oligonucleotides, primers, probes, or antibodies are modified or labeled, for example, but without limitation, by radioactive or immunological labeling. Thus, preferably, the oligonucleotides have modifications in some of their nucleotides, such as, but not limited to, nucleotides having some of their atoms replaced with a radioactive isotope, typically 32P or tritium, nucleotides immunologically labeled, such as with a digoxigenin molecule, and/or immobilized on a membrane. Several possibilities are known in the prior art.

The kit or device of the invention may comprise controls, program instructions, and information necessary to carry out any of the methods of the invention.

Another **aspect** of the invention relates to the use of the kit according to any of the embodiments described above to carry out any of the embodiments of the method of the invention.

### COMPUTER-IMPLEMENTED INVENTION

Another **aspect** of the invention relates to computer-readable storage media comprising program instructions capable of causing a computer to perform the steps of any of the methods of the invention. The invention also extends to computer programs adapted for any processing medium to carry out the methods of the invention. Such programs may take the form of source code, object code, an intermediate source code and object code, for example in partially compiled form, or in any other form suitable for use in implementing the processes according to the invention. The computer programs also encompass cloud-based applications based on this procedure. In particular, the invention encompasses computer programs arranged on or within a carrier. The carrier may be any entity or device capable of supporting the program. When the program is incorporated into a signal that can be carried directly by a cable or other device or medium, the carrier may consist of that cable or other device or medium. Alternatively, the carrier could be an integrated circuit in which the program is included, the integrated circuit being adapted to execute, or to be used in the execution of, the corresponding processes.

For example, the programs could be integrated into a storage medium, such as a ROM, a CD-ROM, or a semiconductor ROM, a USB memory stick, or a magnetic recording medium, for example, a floppy disk or a hard disk. Alternatively, the programs could be supported by a transmissible carrier signal. For example, this could be an electrical or optical signal that could be transported via an electrical or optical cable, by radio, or by any other means. The invention also extends to computer programs adapted so that any processing medium can carry out the methods of the invention. Such programs may take the form of source code, object code, an intermediate source code and object code, for example, in partially compiled form, or in any other form suitable for use in implementing the processes according to the invention. The computer programs also encompass cloud-based applications based on this procedure. Therefore, another aspect of the invention relates to a computer-readable storage medium comprising program instructions capable of causing a computer to perform the steps of any of the methods of the invention.

Another aspect of the invention relates to a transmissible signal comprising program instructions capable of causing a computer to perform the steps of any of the methods of the invention.

In the context of the present invention, the terms "subject," "patient," or "individual" are used here interchangeably to refer to all animals classified as mammals and include, but are not limited to, domestic and farm animals, primates, and humans, e.g., humans, non-human primates, cows, horses, pigs, sheep, goats, dogs, cats, or rodents. Preferably, the subject is a male or female human of any age or race.

Throughout the description and claims, the word "comprising" and its variants are not intended to exclude other technical features, additions, components, or steps. For those skilled in the art, other objects, advantages, and features of the invention will be understood in part from the description and in part from the practice of the invention. The following examples and drawings are provided for illustration purposes and are not intended to limit the present invention.

An "isolated biological sample" includes, but is not limited to, cells, tissues, and/or biological fluids from an organism, obtained by any method known to a person skilled in the art. Preferably, the isolated biological sample is skin or peripheral venous blood.

The terms "one or more" or "at least one" or "several," as used herein, include one and the individualized specification of any number that is more than one, such as two, three, four, five, six, etc.

### EXAMPLES OF THE INVENTION

The present invention is based on the analysis of 740 miRNAs in samples of damaged skin and peripheral blood from patients with non-segmental vitiligo (n=20) and healthy controls (n=10).

After the approval of the protocol by the local Research Ethics Committee, patients were selected based on the following criteria: a) adults; b) with clinically or histologically diagnosed non-segmental vitiligo; c) *naive* to local systemic treatments or after drug washout (2 weeks for topical and 4 weeks for non-biological systemic treatments); d) no other comorbidities or autoimmune diseases, except for Hashimoto's thyroiditis in an euthyroid state.

The severity of vitiligo was assessed using the VASI scale, and the duration of the disease was calculated based on each patient's medical history. Once the various patient consents had been obtained, photographs were taken and skin biopsies were obtained under local anesthesia with a 5 mm punch from a **damaged area (LS)** and other **non- damaged area (NL)** of the skin. In the case of healthy controls, a single skin sample was obtained. **Peripheral venous blood** samples were obtained from all participants.

These samples were analyzed by RT-PCR using the OpenArray and Thermofisher Connet systems. Expression values [cycle threshold (Ct)] were normalized with miRNA expression values by transforming Ct values to -dCt. Missing values were estimated for each gene as 20% of the minimum expression values in all samples. After quality control and *batch* correction, expression differences between groups were analyzed using a nonparametric approach with the Wilcoxon test and considering a significance level of p < 0.05. This yielded different ranked lists of differentially expressed miRNAs. Statistical analyses were performed using the statistical language R (www.r-project.org).

As a result, **90 differentially expressed miRNAs** were identified **in the damaged skin** of patients with **non-segmental vitiligo** compared to healthy volunteers, in whom no significant changes were found (p < 0.05). These miRNAs are identified in **Table 2,** indicating for each case the level of expression in patients with non-segmental vitiligo and in controls, as well as associated p-values.

**Table 2. List of miRNAs with significantly different expression in damaged skin samples from patients with non-segmental vitiligo vs. controls.**

| **miRNAs** | **Vitiligo** | **controls** | **P value** | **Fold-change** |
|---|---|---|---|---|
| hsa.miR.365a.3p | 0.4188956 | 1.1509775 | 0.0060848554701539 | -0.7320819 |
| hsa.miR.1249.3p | 0.3469913 | 0.9169925 | 0.00790001969082206 | -0.5700012 |
| hsa.miR.664a.3p | 0.4534453 | 1.1509775 | 0.00790001969082206 | -0.6975322 |
| hsa.miR.92a.3p | 0.4797487 | 1.1509775 | 0.0095124048725034 | -0.6712288 |
| hsa.miR.106b.5p | 0.4234893 | 1.033985 | 0.00985696008583745 | -0.6104957 |
| hsa.miR.149.5p | 0.4493481 | 1.1509775 | 0.00985696008583745 | -0.7016294 |
| hsa.miR.374a.5p | 0.4299988 | 1.033985 | 0.00985696008583745 | -0.6039862 |
| hsa.miR.374b.5p | 0.4256919 | 1.033985 | 0.00985696008583745 | -0.6082931 |
| hsa.miR.425.5p | 0.4234893 | 1.033985 | 0.00985696008583745 | -0.6104957 |
| hsa.miR.148a.3p | 0.4486787 | 1.1509775 | 0.00992659306922461 | -0.7022988 |
| hsa.miR.26a.5p | 0.4519953 | 1.1509775 | 0.00992659306922461 | -0.6989822 |
| hsa.miR.203a.3p | 0.4345924 | 1.1509775 | 0.0100665773973097 | -0.7163851 |
| hsa.miR.200c.3p | 0.6001628 | 1.1509775 | 0.0101466589313789 | -0.5508147 |
| hsa.miR.99a.5p | 0.4700526 | 1.1509775 | 0.0109464598439017 | -0.6809249 |
| hsa.miR.224.5p | 0.4797487 | 1.033985 | 0.0110234163882214 | -0.5542363 |
| hsa.miR.193b.3p | 0.5323556 | 1.1509775 | 0.0113233290719574 | -0.6186219 |
| hsa.miR.296.5p | 0.4563022 | 1.033985 | 0.0114905238785233 | -0.5776828 |
| hsa.miR.30b.5p | 0.4497927 | 1.033985 | 0.0114905238785233 | -0.5841923 |
| hsa.miR.93.5p | 0.4334783 | 1.033985 | 0.0116482577894378 | -0.6005067 |
| hsa.miR.1271.5p | 0.5212525 | 1.033985 | 0.0119050810662381 | -0.5127325 |
| hsa.miR.148b.3p | 0.5630985 | 1 | 0.0119050810662381 | -0.4369015 |
| hsa.miR.423.3p | 0.5212525 | 1.033985 | 0.0119050810662381 | -0.5127325 |
| hsa.miR.455.3p | 0.5212525 | 1.033985 | 0.0119050810662381 | -0.5127325 |
| hsa.miR.130a.3p | 0.4760962 | 1.033985 | 0.0126729439685062 | -0.5578888 |
| hsa.miR.205.5p | 0.4608958 | 1.033985 | 0.0126729439685062 | -0.5730892 |
| hsa.miR.30c.5p | 0.4782988 | 1.033985 | 0.0126729439685062 | -0.5556862 |
| hsa.miR.29c.5p | 0.5198025 | 1.033985 | 0.0131344295302774 | -0.5141825 |
| hsa.miR.324.5p | 0.5198025 | 1.033985 | 0.0131344295302774 | -0.5141825 |
| hsa.miR.195.3p | 0.4510885 | 0.9509775 | 0.0136013995262562 | -0.499889 |
| hsa.miR.486.5p | 0.3750869 | 1.1323556 | 0.0139107724904636 | -0.7572687 |
| hsa.miR.106b.3p | 0.4908519 | 0.9169925 | 0.0140756679761432 | -0.4261406 |
| hsa.miR.378a.3p | 0.4686456 | 1.033985 | 0.0141713882540123 | -0.5653394 |
| hsa.miR.194.5p | 0.2803896 | 1.0153631 | 0.0144570241220671 | -0.7349735 |
| hsa.miR.494.3p | 0.4241674 | 1.1509775 | 0.0152936533065191 | -0.7268101 |
| hsa.miR.429 | 0.3445789 | 1.0153631 | 0.0162248212813898 | -0.6707842 |
| hsa.miR.204.5p | 0.3856381 | 1.0983706 | 0.0162752337441366 | -0.7127325 |
| hsa.miR.199b.5p | 0.471784 | 1.033985 | 0.0204432159782943 | -0.562201 |
| hsa.miR.192.5p | 0.4732947 | 1.033985 | 0.0210350160709514 | -0.5606903 |
| hsa.miR.34a.5p | 0.5591037 | 1.1509775 | 0.0212610876077463 | -0.5918738 |
| hsa.miR.29b.3p | 0.5497927 | 1.1509775 | 0.0216410138445844 | -0.6011848 |
| hsa.let.7b.3p | 0.6586591 | 1.1509775 | 0.0218556343405187 | -0.4923184 |
| hsa.miR.28.3p | 0.641666 | 1.1509775 | 0.022320268763123 | -0.5093109 |
| hsa.miR.151a.5p | 0.6060522 | 1.1509775 | 0.022626299499965 | -0.5449253 |
| hsa.miR.25.3p | 0.6060522 | 1.1509775 | 0.022626299499965 | -0.5449253 |
| hsa.miR.140.3p | 0.538245 | 1.033985 | 0.0227957869735389 | -0.49574 |
| hsa.miR.154.5p | 0.5299988 | 1.033985 | 0.0227957869735389 | -0.5039862 |
| hsa.miR.93.3p | 0.5826057 | 1.1509775 | 0.0230376672528961 | -0.5683718 |
| hsa.miR.26b.5p | 0.4782988 | 1.1849625 | 0.0231753899516528 | -0.7066637 |
| hsa.miR.99b.3p | 0.5230447 | 1.033985 | 0.0234468127750727 | -0.5109403 |
| hsa.miR.29c.3p | 0.508289 | 1.033985 | 0.0235778133995956 | -0.525696 |
| hsa.miR.191.5p | 0.5797487 | 1.033985 | 0.0247096888099684 | -0.4542363 |
| hsa.miR.24.3p | 0.5797487 | 1.033985 | 0.0247096888099684 | -0.4542363 |
| hsa.miR.532.3p | 0.5797487 | 1.033985 | 0.0247096888099684 | -0.4542363 |
| hsa.miR.361.5p | 0.3934996 | 1.0983706 | 0.0249020630565251 | -0.704871 |
| hsa.miR.16.5p | 0.3971858 | 0.9813781 | 0.0252761920464 | -0.5841923 |
| hsa.miR.629.5p | 0.4645484 | 0.9509775 | 0.0255611128335053 | -0.4864291 |
| hsa.miR.484 | 0.5493481 | 1.033985 | 0.0258471320626969 | -0.4846369 |
| hsa.miR.345.5p | 0.536795 | 1.033985 | 0.0259905502476329 | -0.49719 |
| hsa.miR.125a.5p | 0.6586591 | 1.033985 | 0.0261897379012848 | -0.3753259 |
| hsa.miR.1301.3p | 0.6060522 | 1.033985 | 0.0267908008556994 | -0.4279328 |
| hsa.let.7e.3p | 0.5826057 | 1.033985 | 0.0272531967766466 | -0.4513793 |
| hsa.miR.126.3p | 0.5756516 | 1.033985 | 0.0272531967766466 | -0.4583334 |
| hsa.miR.151b | 0.5908519 | 1.033985 | 0.0272531967766466 | -0.4431331 |
| hsa.miR.218.5p | 0.5908519 | 1.033985 | 0.0272531967766466 | -0.4431331 |
| hsa.miR.326 | 0.5756516 | 1.033985 | 0.0272531967766466 | -0.4583334 |
| hsa.miR.151a.3p | 0.6323556 | 1.033985 | 0.0273949730896586 | -0.4016294 |
| hsa.miR.328.3p | 0.5674053 | 1.033985 | 0.0274079127034367 | -0.4665797 |
| hsa.miR.125b.5p | 0.6171553 | 1.033985 | 0.0275594988114761 | -0.4168297 |
| hsa.miR.221.3p | 0.6171553 | 1.033985 | 0.0275594988114761 | -0.4168297 |
| hsa.miR.497.5p | 0.5871993 | 1.033985 | 0.0275594988114761 | -0.4467857 |
| hsa.miR.17.5p | 0.3808715 | 1.1323556 | 0.0277322967875613 | -0.7514841 |
| hsa.miR.23a.3p | 0.6153631 | 1.033985 | 0.0295585835294018 | -0.4186219 |
| hsa.miR.758.3p | 0.6153631 | 1.033985 | 0.0295585835294018 | -0.4186219 |
| hsa.miR.199a.5p | 0.5334783 | 0.9169925 | 0.0297625165405284 | -0.3835142 |
| hsa.miR.425.3p | 0.6230447 | 1.033985 | 0.0305609631681744 | -0.4109403 |
| hsa.miR.128.3p | 0.5604513 | 0.9169925 | 0.0307450347589932 | -0.3565412 |
| hsa.miR.10b.5p | 0.594949 | 1.150978 | 0.0310431524724214 | -0.556029 |
| hsa.miR.214.3p | 0.5894019 | 0.9169925 | 0.0317010306854173 | -0.3275906 |
| hsa.miR.20a.5p | 0.3803112 | 1.0153631 | 0.0328304964085471 | -0.6350519 |
| hsa.miR.335.5p | 0.5256919 | 1.06797 | 0.0343873103418464 | -0.5422781 |
| hsa.miR.15a.5p | 0.60281 | 1.150978 | 0.0356271004877229 | -0.548168 |
| hsa.miR.1296.5p | 0.5212525 | 1.06797 | 0.0368788018411012 | -0.5467175 |
| hsa.miR.29b.2.5p | 0.5749821 | 1.1509775 | 0.0379607574725193 | -0.5759954 |
| hsa.miR.378a.5p | 0.538245 | 1.033985 | 0.0385047628410804 | -0.49574 |
| hsa.miR.34a.3p | 0.506193 | 1.033985 | 0.0395986235887438 | -0.527792 |
| hsa.miR.195.5p | 0.4782988 | 0.9813781 | 0.0431832773036339 | -0.5030793 |
| hsa.miR.99a.3p | 0.6586591 | 1.1509775 | 0.0436835892303467 | -0.4923184 |
| hsa.miR.652.3p | 0.5738594 | 1.0983706 | 0.0439104994182379 | -0.5245112 |
| hsa.miR.188.5p | 0.5212525 | 1.0983706 | 0.04775347478001 | -0.5771181 |
| hsa.miR.221.5p | 0.597806 | 1.033985 | 0.0499242840396976 | -0.436179 |

A series of miRNAs were also identified that were differentially expressed in **plasma samples** from patients with non-segmental vitiligo compared to controls. The results are shown below in **Table 3** and correspond to the following miRNAs: **miR-16-5p, miR-92a-3p, miR-486-5p, miR-221-3p, miR-23a-3p, and miR-652-3p.**

**Table 3. List of miRNAs with significantly different expression in plasma samples from patients with non-segmental vitiligo vs. controls.**

| **miRNAs** | **Vitiligo** | **controls** | **P value** | **Fold Change** |
|---|---|---|---|---|
| hsa.miR.16.5p | 0.4372043 | 1.1509775 | 0.0210212271224784 | -0.7137732 |
| hsa.miR.486.5p | 0.546574 | 1.150978 | 0.0271089711585456 | -0.604404 |
| hsa.miR.652.3p | 2.189375 | 1.098371 | 0.0280183816317403 | 1.091004 |
| hsa.miR.221.3p | 1.931383 | 1.06797 | 0.0449492898576499 | 0.863413 |
| hsa.miR.92a.3p | 0.5618522 | 1.1509775 | 0.0452027580684779 | -0.5891253 |
| hsa.miR.23a.3p | 2.329712 | 1.215363 | 0.0464745717631752 | 1.114349 |

It was verified that the six miRNAs dysregulated in plasma in patients with non-segmental vitiligo were also found in the panel of under-expressed miRNAs in damaged skin in **Table 1,** confirming that the panel of miRNAs of the invention is selective in representing the degree of systemic involvement in patients with vitiligo.

### References

1. Montilla AM, Gómez-García F, Gómez-Arias PJ, Gay-Mimbrera J, Hernández-Parada J, Isla-Tejera B, Ruano J. Scoping Review on the Use of Drugs Targeting JAK/STAT Pathway in Atopic Dermatitis, Vitiligo, and Alopecia Areata. Dermatol Ther (Heidelb), 2019.
2. Zhang L, Chen S, Kang Y, Wang X, Yan F, Jiang M, Wang Q, Liu Z, Zhang C, Xiang L. Association of Clinical Markers With Disease Progression in Patients With Vitiligo From China. JAMA Dermatol. 2020 Mar 1;156(3):288-295. doi: 10.1001/jamadermatol.2019.4483. PMID: 31968061; PMCID: PMC6990655.

## Claims

1. *In vitro* use of the expression levels of the miRNA in Table 1 simultaneously as biomarkers for diagnosing and/or predicting non-segmental vitiligo.

2. An *in vitro* method for obtaining useful data for diagnosing and/or predicting non-segmental vitiligo, comprising measuring the expression levels of all miRNAs in Table 1 in a biological sample of previously isolated damaged skin tissue or blood, serum, or plasma.

3. An *in vitro* method for diagnosing and/or predicting non-segmental vitiligo comprising:
a) measuring the expression levels of all miRNAs in Table 1 in a previously isolated sample of damaged skin tissue,
b) comparing the values obtained with those of a reference sample obtained from healthy control individuals, and
c) classifying the individual into the group of individuals suffering from severe non-segmental vitiligo when the miRNAs in step a) are differentially down-expressed compared to the reference sample from healthy control individuals.

4. An *in vitro* method for diagnosing and/or predicting non-segmental vitiligo comprising:
a) measuring the expression levels of all miRNAs in Table 1 in a previously isolated blood, serum, or plasma sample,
b) comparing the values obtained with those of a reference sample obtained from healthy control individuals, and
c) classifying the individual in the group of individuals suffering from severe non-segmental vitiligo when the miRNAs in step a) hsa.miR.16.5p, hsa.miR.486.5p, and hsa.miR.92a.3p are differentially down-expressed and hsa.miR.652.3p, hsa.miR.221.3p, and hsa.miR.23a.3p are differentially over-expressed compared to reference samples from healthy control individuals.

5. Use of miRNAs in Table 1 in a method for predicting the therapeutic response of a treatment for non-segmental vitiligo.

6. Method for predicting the therapeutic response of a patient with non-segmental vitiligo to a treatment for non-segmental vitiligo, comprising:
a) measuring the expression levels of one or more of the miRNAs in Table 1 in a biological sample previously isolated from a patient with non-segmental vitiligo prior to administration of the treatment for vitiligo,
b) measuring the expression levels of the same miRNAs from step a) in a biological sample previously isolated from the same patient with non-segmental vitiligo after administration of the treatment for vitiligo,
c) comparing the values obtained in steps a) and b), and
d) classifying the individual in the group of individuals responding to treatment when the miRNAs from step b) are differentially expressed with respect to the samples from step a).

7. Method for predicting the therapeutic response to a treatment for non-segmental vitiligo in patients with non-segmental vitiligo, comprising:
a) measuring the expression levels of one or more of the miRNAs in Table 1 in biological samples previously isolated from a statistically significant number of patients with non-segmental vitiligo prior to the administration of the treatment for vitiligo,
b) measuring the expression levels of the same miRNAs as in step a) in a biological sample previously isolated from the same patients with non-segmental vitiligo after administration of the treatment for vitiligo,
c) comparing the values obtained in steps a) and b), and
d) classifying the treatment for non-segmental vitiligo as effective when the miRNA(s) from step b) are differentially expressed with respect to the samples from step a) in a statistically significant number of patients with non-segmental vitiligo undergoing treatment.

8. The method according to any of claims 6 to 7, **characterized in that** the previously isolated biological sample is selected from damaged skin, blood, plasma, and serum.

9. The method according to the previous claim, **characterized in that** the previously isolated biological sample is damaged skin tissue.

10. The method according to any of claims 2 to 4 and 6 to 9, wherein said result can be obtained by:
(i) a method of generating miRNA profiles, such as a microarray, and/or
(ii) a method comprising PCR (polymerase chain reaction), such as real-time PCR or RT-PCR, and/or
(iii) a Northern Blot and/or
(iv) an immunoassay, such as an immunohistochemistry method or an ELISA-based method.

11. A kit or device comprising at least one or more oligonucleotides capable of hybridizing to all miRNAs in Table 1 simultaneously under stringent conditions.

12. Use of the kit according to the preceding claim in a method according to any of claims 2-4 and 6-10.

## Amended claims

### Amended claims under Art. 19.1 PCT

1. *In vitro* use of the expression levels of the miRNA in Table 1 simultaneously as biomarkers for diagnosing and/or predicting non-segmental vitiligo.

2. An *in vitro* method for obtaining useful data for diagnosing and/or predicting non-segmental vitiligo, comprising measuring the expression levels of all miRNAs in Table 1 in a biological sample of previously isolated damaged skin tissue or blood, serum, or plasma.

3. An *in vitro* method for diagnosing and/or predicting non-segmental vitiligo comprising:
a) measuring the expression levels of all miRNAs in Table 1 in a previously isolated sample of damaged skin tissue,
b) comparing the values obtained with those of a reference sample obtained from healthy control individuals, and
c) classifying the individual into the group of individuals suffering from severe non-segmental vitiligo when the miRNAs in step a) are differentially down-expressed compared to the reference sample from healthy control individuals.

4. An *in vitro* method for diagnosing and/or predicting non-segmental vitiligo comprising:
a) measuring the expression levels of all miRNAs in Table 1 in a previously isolated blood, serum, or plasma sample,
b) comparing the values obtained with those of a reference sample obtained from healthy control individuals, and
c) classifying the individual in the group of individuals suffering from severe non-segmental vitiligo when the miRNAs in step a) hsa.miR.16.5p, hsa.miR.486.5p, and hsa.miR.92a.3p are differentially down-expressed and hsa.miR.652.3p, hsa.miR.221.3p, and hsa.miR.23a.3p are differentially over-expressed compared to reference samples from healthy control individuals.

5. Use of miRNAs in Table 1 in a method for predicting the therapeutic response of a treatment for non-segmental vitiligo.

6. Method for predicting the therapeutic response of a patient with non-segmental vitiligo to a treatment for non-segmental vitiligo, comprising:
a) measuring the expression levels of all miRNAs in Table 1 in a biological sample previously isolated from a patient with non-segmental vitiligo prior to administration of the treatment for vitiligo,
b) measuring the expression levels of the same miRNAs as in step a) in a biological sample previously isolated from the same patient with non-segmental vitiligo after administration of the treatment for vitiligo,
c) comparing the values obtained in steps a) and b), and
d) classifying the individual in the group of individuals responding to treatment when the miRNAs from step b) are differentially expressed with respect to the samples from step a).

7. Method for predicting the therapeutic response to a treatment for non-segmental vitiligo in patients with non-segmental vitiligo, comprising:
a) measuring the expression levels of all miRNAs in Table 1 in biological samples previously isolated from a statistically significant number of patients with non-segmental vitiligo prior to administration of the treatment for vitiligo,
b) measuring the expression levels of the same miRNAs as in step a) in a biological sample previously isolated from the same patients with non-segmental vitiligo after administration of the treatment for vitiligo,
c) comparing the values obtained in steps a) and b), and
d) classifying the treatment for non-segmental vitiligo as effective when the miRNAs from step b) are differentially expressed with respect to the samples from step a) in a statistically significant number of patients with non-segmental vitiligo undergoing treatment.

8. The method according to any of claims 6 to 7, **characterized in that** the previously isolated biological sample is selected from damaged skin, blood, plasma, and serum.

9. The method according to the previous claim, **characterized in that** the previously isolated biological sample is damaged skin tissue.

10. The method according to any of claims 2 to 4 and 6 to 9, wherein said result can be obtained by:
(i) a method of generating miRNA profiles, such as a microarray, and/or
(ii) a method comprising PCR (polymerase chain reaction), such as real-time PCR or RT-PCR, and/or
(iii) a Northern Blot and/or
(iv) an immunoassay, such as an immunohistochemistry method or an ELISA-based method.

11. A kit or device consisting of one or more oligonucleotides capable of hybridizing with all the miRNAs in Table 1 simultaneously under stringent conditions.

12. Use of the kit according to the preceding claim in a method according to any of claims 2-4 and 6-10.

Statement under Art. 19.1 PCT
These amendments are submitted in order to overcome the objection regarding lack of unity of invention raised during the international search and to overcome the objection to the novelty of claim 11 directed to the kit of the invention.
